# EUROPEAN PATENT APPLICATION

(11) **EP 2 206 462 A1**
(43) Date of publication of application: **14.07.2010**
(21) Application number: 09150474.6
(22) Date of filing: 13.01.2009
(51) Int. Cl.: A61B 5/00

(54) **A non-invasive chemical sensor, a skin patch, a packaging material and a monitoring system using the same**

(71) Applicant: Nederlandse Organisatie voor toegepast -natuurwetenschappelijk onderzoek TNO, 2628 VK Delft (NL)
(72) Inventor: Van den Brand, Jeroen, 5053 AT Goirle (NL); Sharpe, Ruben Bernardus Alfred, 2651 PC Berkel en Rodenrijs (NL); Koetse, Marinus Marc, 5644 EE Eindhoven (NL)
(74) Representative: Hatzmann, Martin

(57) **Abstract**

The invention relates to a non-invasive chemical sensor 10 for detecting a substance present on an object, comprising a surface conceived to be arranged in contact with the object, wherein the surface is formed by a porous material comprising a chemical reagent arranged to undergo a chemical interaction with the substance. A surface area of the sensor 10 may be divided into a suitable number of compartments, which may be arranged as a matrix having a number of columns C1,..,.Cn and a number of rows R1,..,Rn. It will be appreciated that the compartments, alternatively, may be irregularly positioned and/or they may have an irregular size. The compartments C1,..,.Cn may be arranged with the same reagent. In this case it is possible to differentiate signals originating from different compartments by means of suitable detection and data processing.

## Description

### FIELD

The invention relates to a non-invasive chemical sensor for detecting and/or monitoring presence of a substance on a surface. In particular, the invention relates to a non-invasive chemical sensor for detecting and/or monitoring presence and/or amount of a compound in a body fluid excreted by skin.

The invention further relates to a skin patch, a packaging material and a monitoring system using a non-invasive chemical sensor as is set forth in the opening paragraph.

### BACKGROUND

An embodiment of a non-invasive chemical sensor is known from WO 2007/146047. The known non-invasive chemical sensor is arranged for monitoring a subject's glucose level. The known sensor comprises a sweat-permeable layer for conducting sweat generated by a skin area towards an internal reservoir wherein suitable measurements are carried out. For example, glucose concentration may be determined by performing suitable normalizations regarding sweat rates. Additionally, a separate detector may be used for determining the amount of the sweat analyte in the reservoirs.

It is a disadvantage of the known chemical sensor that a sophisticated device architecture is required for enabling both collection of the analyte and measurement of glucose level therein.

Another embodiment of a sensor as is set forth in the opening paragraph is known from WO 00/57177. The known non-invasive transdermal sensor comprises a wet chemistry component and a dry chemistry component. The wet chemistry component is arranged in contact with a fluid conceived to be analyzed and comprises a liquid transfer medium for extracting an analyte. The wet chemistry component is arranged to transport the analyte towards the dry chemistry component. The dry chemistry component comprises a reagent system for interacting with the analyte for generating an indication that a particular substance is present in the analyte. For example, colour signalling is contemplated. While during storage the wet chemistry component is physically separated from the dry chemistry component, in use they have to be brought in a direct continuous contact, so that the wet chemistry component can transfer the analyte to the dry chemistry component for interacting with a reagent stored therein. In the known sensor it is required that a substantially continuous and uniform wetting of the dry chemical component is enabled. For this purpose the known sensor comprises housing enabling a perfect alignment between the wet chemistry component and a dry chemistry component in use.

The above sensor has a disadvantage that it has to be manipulated in use for transferring analyte from the wet chemical component to the dry chemical component for enabling analysis. Such manipulation may not be suitable for monitoring purposes. In addition, the known sensor has a disadvantage that its functioning is dependent upon alignment between the dry chemistry component and the wet chemistry component so that the dry chemistry component has to be preserved in direct and continuous contact with the wet chemistry component.

### SUMMARY OF THE INVENTION

It is an object of the invention to provide a non-invasive chemical sensor for detecting and/or monitoring presence of a chemical substance on an object, which is simple, reliable and which can be manufactured with relatively low production costs.

To this end, a non-invasive chemical sensor for detecting a substance present on an object according to an aspect of the invention comprises a surface conceived to be arranged in contact with the object, wherein the surface is formed by a porous material comprising a chemical reagent arranged to undergo a chemical interaction with the substance.

It is found to be advantageous to provide the non-invasive chemical sensor with a contact surface which is porous and wherein suitable chemical reagent is embedded. The chemical reagent may be provided in all or in some of the pores. For example, for medical or paramedical applications, it may be preferable to provide the non-invasive chemical sensor on a person's skin for a prolonged amount of time. The porous contact material allows breathing of a skin area underneath the sensor thereby reducing risk of erythema.

Secondly, due to providing a contact layer with a porous material comprising reagent arranged at least in some of the pores, there is no necessity for enabling fluid transport towards a further analytic location within the sensor, like a reservoir or dry chemistry component. Next, due to a large surface area of a porous structure a reaction possibility between the reagent present in the pores and the analyte is increased. This increases reliability of the sensor as a whole. It will be appreciated that when the reagent is arranged to change colour or opacity and when the porous material is provided with reduced thickness, a change in colour or opacity of the reagent may be used for qualitative interpretation. For example, the sensor may be used as alarming means for providing suitable perceptive signalling when a (hazardous) substance is detected.

In an embodiment of the sensor according to the invention the surface conceived to contact the object extends over an area, the porous material comprising a plurality of compartments arranged over the area, wherein the chemical reagent is arranged in the compartments.

According to the present aspect of the invention the sensor may be arranged to enable position-sensitive analysis. For example, the area of the surface conceived to contact the object may be implemented as a suitable arrangement of compartments, for example a matrix of compartments. The compartments may be arranged to be substantially isolated from each other. According to one embodiment, the compartments may be provided with the same reagent so that a suitable position-dependent analysis is enabled. This may be of a particular interest for increased surface areas of the sensor, for example for areas of about a few square decimetres. Such application, for example, may relate to skin monitoring during or post external radiotherapy, wherein a radiation beam interacting with the skin may induce position-dependent injury. Alternatively, enlarged sensors may be used for improving statistics of data collection, which may be advantageous when such sensor provides input data for a monitoring system.

Alternatively or additionally, different compartments of the sensor may be provided with different reagents for enabling targeted detection and/or monitoring of different substances possibly present on a surface of the object. This has an advantage that specificity of the non-invasive chemical sensor according to the invention is increased.

In a further embodiment of a sensor according to the invention, the sensor further comprises a detector arranged to provide a signal in response to the chemical reagent undergoing a chemical reaction with said substance.

The non-invasive chemical sensor according to the invention may be implemented as a layer of porous material, provided with a reagent, on top of which a suitable detector is provided. Preferably, both the porous material and a detector structure are implemented as suitable thin layers. Such structure may have low sensitivity for misalignments during manufacturing and use, for example with respect to a lateral shift between a layer of porous material and a layer of a detector structure, which substantially reduces manufacturing costs of the sensor. For example, the sensor may be produced using ink-jet printing for depositing suitable reagents in the porous material.

In accordance with a still further embodiment of the sensor according to the invention, the detector comprises an active foil arranged on a back surface of the porous material.

It will be appreciated that in case when the porous material is substantially homogeneous with respect to the reagent type and concentration distribution along it area, the detector may relate to a structure which collects data from substantially the whole area of the porous material. In case when the porous material is heterogeneous, for example when it comprises compartments, the detector may comprise a suitable plurality of detector elements arranged in an array spatially matching the compartments. It is found that by combining plural components in detector and by providing a homogenous sensor acquisition of information relating to excretion may be enabled.

It will be appreciated that a plurality of operational mechanisms of the sensor according to the invention is envisaged. For example, the reagent may operate using one of the following principles: optical, calorimetric, electric. In this case the detector is arranged to be responsive to a corresponding change in a property of the reagent.

Preferably, the reagent and the detector operate using change in an optical property of the reagent, for example a suitable change in colour. In this case, for example, a bottom layer of the sensor may be provided with the porous material carrying reagent and a top layer which may be implemented as a OLED layer. In addition, a suitable detector structure may comprise an optical filter cooperating with a photosensor, for example a photodiode, which may be organic. For a sensor arranged with compartments, such detector structure may be provided per compartment.

In operation, reflected light from the porous layer may pass through a suitable filter, for example a colour filter for extracting information regarding a colour change in the reagent, which is subsequently monitored by the photosensor. In an embodiment, when the sensor comprises a layered structure, the photosensor may comprise a photodiode film.

In another embodiment of the sensor according to the invention, the chemical reagent may be arranged to change an electrical characteristic upon reaction with the substance. In this case the detector may comprise electrodes for detecting such change.

For example, pursuant to a reaction between the reagent and the substance, the former may change an electrical characteristic, for example capacitance or impedance, which can be easily detected by two electrodes arranged in the porous material.

It will be appreciated that when a homogeneous sensor is provided, the electrodes may be positioned at suitable locations across the sensor. When the sensor is of a considerable area, the electrodes may be located to encompass the whole surface area of the sensor or a portion thereof for indicating that a change has occurred. Alternatively, when the sensor is provided with compartments, the electrodes may be provided per compartment for enabling position-sensitive detection.

Preferably, for improving a signal to noise ratio, the electrodes are provided at least partially in the porous material. However, for application when the porous material is wetted considerably, the electrodes may be provided on an outer surface of the porous material without substantial penetration into it.

In a still further embodiment of the sensor according to the invention, the chemical reagent may be arranged to generate gas or heat upon undergoing a chemical reaction with the substance.

It will be appreciated that a type of the reagent to be provided in the pores of the porous material of the sensor may be suitably optimized regarding a type of a chemical substance which is conceived to be detected or monitored. Different types of possible chemical interactions between the reagent and the substance are contemplated. It may be that for a certain class of substances particular phenomena may be especially profound. For example, the reagent may be conceived to undergo a chemical reaction with the targeted chemical species to generate a gas or heat or to provide any other measurable effect, which can be detected and/or measured by the detector.

In a still further embodiment of the sensor according to the invention, at least the surface conceived to be arranged in contact with the object is flexible.

This feature improves surface contact between the porous material and the object for non-coplanar areas of the object. In particular, this feature improves wearable properties of the sensor when it is arranged on a patient. As a result, movement of the patient does not lead to de-attachment of the sensor on one hand, and does not provide false positive data due to movement of the patient, on the other hand.

In a still further embodiment of the sensor according to the invention, at least a portion of the surface conceived to be arranged in contact with the object is adhesive.

Preferably, the sensor according to the invention does not require additional means for fastening it on the object. For medical or paramedical applications, like monitoring of a vital sign, it is preferable that the sensor is self-adhering. Preferably, the porous material is used to stick the sensor to the object. When the sensor is provided on a patient, the adhesive porous material enables skin breathing under the sensor which mitigates skin irritation.

In a still further embodiment of the sensor according to the invention, at least a surface the porous material conceived to contact the object is arranged with pores having different dimensions.

This feature is based on the insight that by providing pores with different dimensions, different chemical species present on a surface of the object can be detected. For example, the pores may be dimensioned so that pre-determined target species may penetrate the pores for contacting the reagent. In this case there is no need providing the reagent with specificity per type of species. The porous material in this case will act as a barrier transporting molecules of pre-determined sizes into its internal volume. By arranging the compartments with different pore sizes different target species may be detected at one time. However, it will be appreciated that it is also possible to provide the porous material with sub-regions having different pore sizes without arranging such sib-regions into compartments.

The invention still further relates to a skin patch comprising a sensor as is set forth in the foregoing. For example, the skin patch may comprise a suitable carrier, or, alternatively, it may comprise a suitable peel-off layer for protecting the surface of the sensor conceived to be positioned in contact with the object prior to use.

In a particular embodiment, the skin patch comprises a reagent which is arranged to undergo chemical reaction with a skin excretion product. For example, the reagent may be targeting glucose, a suitable hormone, or any other chemical element whose presence in the excretion fluid should be determined. For example, the reagent may be targeting alcohol, which may be used for an instant alcohol control. It will be appreciated that the term excretion fluid encompasses excretion liquids and excretion gases.

The invention still further relates to a packaging material comprising a sensor as is described with reference to the foregoing. It is found to be advantageous to provide a packaging material comprising a chemical sensor. For example, food may be wrapped in such packaging material for allowing due alarming when the food is of reduced quality.

The invention still further relates to a monitoring system for monitoring of a vital sign comprising a sensor as is set forth with reference to the foregoing.

These and other aspects of the invention will be discussed in more detail with reference to drawings, wherein like reference numerals refer to like elements. It will be appreciated that the drawings are presents for illustrative purposes and may not be used for limiting the scope of the appended claims.

### BRIEF DESCRIPTION OF THE DRAWINGS

Figure 1 presents a schematic view of an embodiment of a non-invasive chemical sensor according to an aspect of the invention.
Figure 2 presents a schematic view of an embodiment of a monitoring system according to the invention.

### DETAILED DESCRIPTION OF THE DRAWINGS

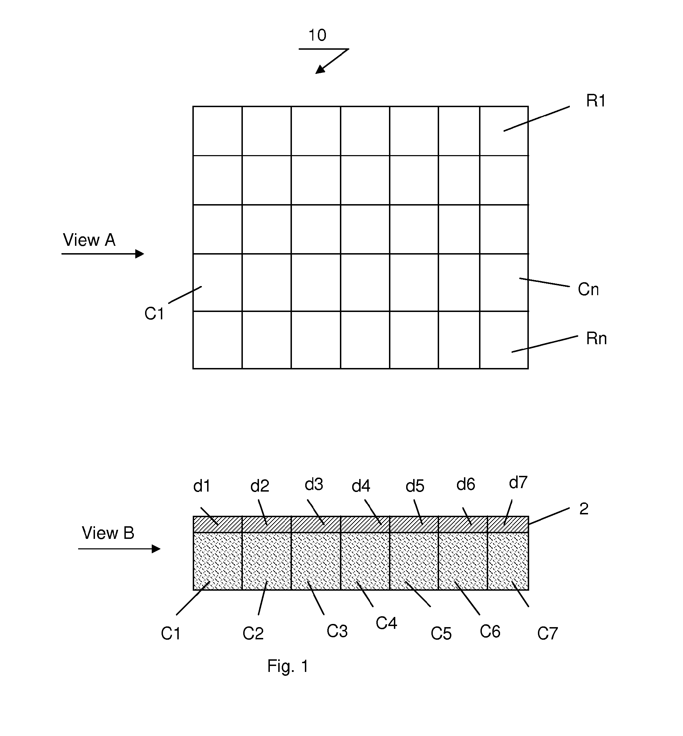

Figure 1 presents a schematic view of an embodiment of a non-invasive chemical sensor according to an aspect of the invention. The non-invasive chemical sensor 10 is schematically depicted in a top view (view A) and in a cross-sectional view (view B). Referring to view A, a surface area of the sensor may be divided into a suitable number of compartments, which may be arranged as a matrix having a number of columns C1,..,Cn and a number of rows R1,..,.Rn. It will be appreciated that the compartments, alternatively, may be irregularly positioned and/or they may have an irregular size.

Compartments may be advantageous in cases when instead of an average signal from the whole sensor area a position dependent signal is desirable. For example, one may need to compare different areas on an object regarding presence and/or amount of a chemical species. In particular, when for the object a living organism is selected, one may need to establish location specific excretion data. By dividing the sensor into a suitable number of compartments such location sensitive data acquisition is enabled.

View B presents a cross-sectional view of the sensor. The sensor is arranged as a layered structure, wherein the porous, preferably adhesive layer is arranged at the bottom of the stack. The porous layer comprises compartments C1,..,.Cn wherein one or more suitable reagents may be provided.

It will be appreciated that the compartments C1,..,.Cn may be arranged with the same reagent. In this case it is possible to differentiate signals originating from different compartments by means of suitable detection and data processing. Those skilled in the art appreciate possible detector-reagent combinations and device architecture suitable for such detection. Although embodiments of possible technology are known to the skilled artisan, for example from WO 00/57177, the invention may be practiced in any particular form enabling signalling of presence of a substance by means of a reaction product of the reagent, or change in the reagent condition with a subsequent signal generation by an electronic, optical or other unit pursuant to such detection.

Alternatively, it is possible to vary a pore size over a surface area of the porous material. Preferably, the pore size is adjusted in accordance with a dimension of a molecule of a substance conceived to be detected. In particular, for simultaneous detection of a variety of substances different pore sizes may be provided for different compartments. In this case the porous material serves as a diffusion barrier for molecules, which are greater in size than the pore dimension. In this case the same reagent may be used for detecting a variety of chemical species, which may be advantageous.

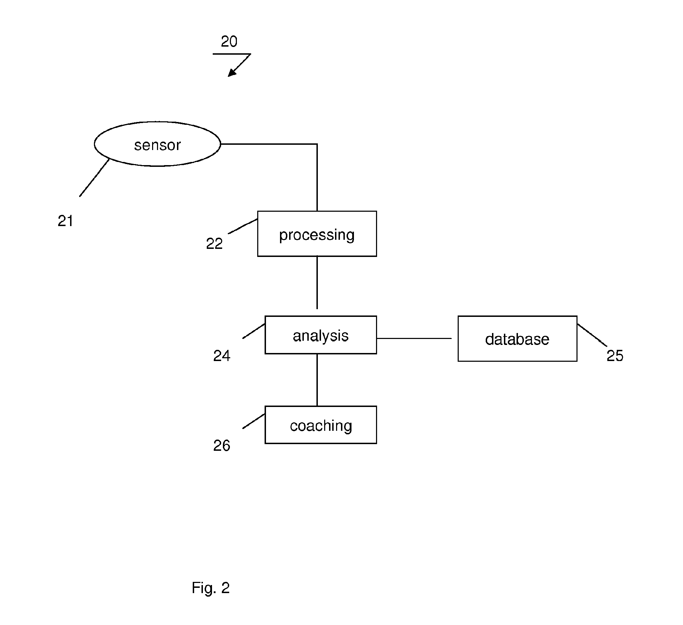

Figure 2 presents a schematic view of an embodiment of a monitoring system according to the invention. The monitoring system 20 may comprise a non-invasive chemical sensor 21 as is discussed with reference to the foregoing. The sensor 21 may be arranged to signal presence of one or more chemical species, for example when it is positioned in contact with a patient skin. Alternatively, the sensor 21 may be arranged for use in industrial applications, for example for examining chemical purity of a surface.

When a chemical species penetrates into a body of a porous material provided with a reagent, the sensor 21 produces a signal indicative that an interaction between the reagent and a chemical species has taken place. For example, when the sensor 21 operates using an electrical principle, for example, a change in impedance, a suitable voltage or a voltage change may be measured by the detector 22, which is preferably integrated in the sensor. The signal from the detector 22 may be forwarded to a signal filtering and processing unit 23 and may be fed into a logic unit 24 for analysis. Preferably, the logic unit 24 operates using suitable databases 25 comprising empiric or theoretical data. For example, the database 25 may relate to calculated or tabulated values of glucose level or hormone level as a function of a type of activity or disorder. When the signal from the sensor is analyzed by the logic unit 24 and is compared to a reference value provided by the database 25, a coaching feed-back may be provided by the coaching unit 26. For example, a message may be generated for suitably advising a patient.

The monitoring system 21 according to the invention may be particularly suitable for life-style management, including coaching during rehabilitation or fitness and may comprise all necessary algorithms and self-teaching models for enabling a proper functioning thereof. It will be appreciated that various implementations of a monitoring system based on a detection of a vital sign are known per se. The monitoring system 21 may utilize known functionalities implementing automatic coaching or advising.

The advantage of the monitoring system 21 according to the invention is that monitoring data is collected using a simple yet reliable sensor which enables data acquisition during a prolonged amount of time with high efficiency due to simplified and shortened path for a species between the analyte and the reagent. By providing the reagent in a porous material interaction probability between the species and the reagent is increased which enables detection of trace elements with high reliability. In addition, the porous material improves skin conditioning, as skin underneath the sensor may still communicate with ambient surroundings, which reduces skin irritation.

It will be appreciated that while specific embodiments of the invention have been described above, that the invention may be practiced otherwise than is described without departing the appended claims. In addition, isolated features discussed with reference to different figures may be combined.

## Claims

1. A non-invasive chemical sensor for detecting a substance present on an object, comprising a surface conceived to be arranged in contact with the object, wherein the surface is formed by a porous material comprising a chemical reagent arranged to undergo a chemical interaction with the substance.

2. A sensor according to claim 1, wherein the surface extends over an area, the porous material comprising a plurality of compartments arranged over the area, wherein the chemical reagent is arranged in the compartments.

3. A sensor according to claim 1 or 2, further comprising a detector arranged to provide a signal in response to the chemical reagent undergoing a chemical reaction with said substance.

4. A sensor according to claim 3, wherein the detector comprises an active foil arranged on a back surface of the porous material.

5. A sensor according to claim 4, the chemical reagent comprising a chemo-luminescent material, wherein the detector comprises a photodiode film.

6. A sensor according to any one of the preceding claims 1-4, wherein the chemical reagent is arranged to change colour upon interaction with the substance.

7. A sensor according to claim 6, wherein the detector comprises a light source arranged to illuminate the porous material, an optical filter arranged to filter specific wavelengths from light reflected from the porous material and a photosensor for generating a signal in response to light passing the optical filter.

8. A sensor according to any one of the preceding claims 1-4, wherein the chemical reagent is arranged to change an electrical characteristic upon reaction with the substance, the detector comprising electrodes for detecting such change.

9. A sensor according to claim 8, when dependent on claim 2, wherein each compartment is provided with the electrodes.

10. A sensor according to claim 9, wherein respective sets of the electrodes are at least partially arranged in the porous material across the compartments.

11. A sensor according to any one of the preceding claim 1 - 4, wherein the chemical reagent is arranged to generate gas or heat upon undergoing a chemical reaction with the substance.

12. A sensor according to any one of the preceding claims, wherein at least the surface conceived to be arranged in contact with the object is flexible.

13. A sensor according to any one of the preceding claims, wherein at least a portion of the surface conceived to be arranged in contact with the object is adhesive.

14. A sensor according to any one of the preceding claims, wherein at least a surface the porous material conceived to contact the object is arranged with pores having different dimensions.

15. A sensor according to claim 14, wherein pores are dimensioned in accordance with a dimension of a molecule of a compound conceived to be detected.

16. A skin patch comprising a sensor according to any one of the preceding claims.

17. A skin patch according to claim 16, wherein the chemical reagent is arranged to undergo chemical reaction with a skin excretion product.

18. A packaging material comprising a sensor according to any one of the preceding claims 1-15.

19. A monitoring system for monitoring of a vital sign comprising a sensor according to any one of the preceding claims 1-15.
